Europäisches Patentamt

European Patent Office

Office européen des brevets

(11) Veröffentlichungsnummer: **0 372 662**
**A2**

## (12) EUROPÄISCHE PATENTANMELDUNG

(21) Anmeldenummer: 89203347.3

(22) Anmeldetag: 06.12.89

(51) Int. Cl.5: **A61B 17/58**

(30) Priorität: 08.12.88 DE 3841286

(43) Veröffentlichungstag der Anmeldung:
**13.06.90 Patentblatt 90/24**

(84) Benannte Vertragsstaaten:
**GR**

(71) Anmelder: **Laabs, Walter, Dr. med.**
**Oestringer Strasse 64**
**D-2948 Grafschaft-Schortens 1(DE)**

Anmelder: **Appel, Hans-Günter, Prof. Dr.**
**Auenweg 2**
**D-2948 Schortens 3 Accum(DE)**

(72) Erfinder: **Laabs, Walter, Dr. med.**
**Oestringer Strasse 64**
**D-2948 Grafschaft-Schortens 1(DE)**
Erfinder: **Appel, Hans-Günter, Prof. Dr.**
**Auenweg 2**
**D-2948 Schortens 3 Accum(DE)**

(74) Vertreter: **Siewers, Gescha, Dr.**
**Rechtsanwälte Dres. Harmsen, Utescher pp.**
**Adenauerallee 28**
**D-2000 Hamburg 1(DE)**

(54) **Osteosyntheseimplantate.**

(57) Die Erfindung betrifft Osteosyntheseimplantate und ist dadurch gekennzeichnet, daß diese als Niet, insbesondere Blindniet und als Kompressionsplatte mit Noppen oder Vorsprüngen auf der dem Knochen zugewandten Seite ausgebildet sind.

EP 0 372 662 A2

## Osteosyntheseimplantate

Bei der operativen Frakturenbehandlung und der Wiederherstellungschirurgie am Knochen läßt sich eine stabile Osteosynthese durch Kompression oder durch intramedulläre Kraftträger, wie Marknägel, erreichen. Während es spezifische Indikationen für beide Verfahren gibt, können bei gewissen Frakturen auch durch beide Methoden einwandfreie Ergebnisse erzielt werden. Es ist schon seit langem bekannt, daß gut durchblutete Knochenfragmente unter Druck so ruhig gestellt werden können, daß Scher- und Torsionskräfte neutralisiert werden und daß es dann nicht zu einer Resorption der Fragmentenden, sondern zu einer direkten ossären Heilung ohne Kallusbildung kommen soll. Bei einer Ruhestellung der Fraktur unter Druck erfolgt die Stabilisierung z.B. mit Kompressionsplatten; die intrafragmentäre Kompression wirkt flächenhaft, d.h. auf der ganzen Ausdehnung der Frakturflächen und wird mittels Zugschrauben gesichert. Standardkompresssionsplatten und Spongiosa-oder Corticaliszugschrauben weisen entsprechend dem Frakturtyp und der Größe des verletzten Knochens unterschiedliche Dimensionen auf. Bei einer Standardmethode zur Fixation von Femurschaftfrakturen wird beispielsweise 1 cm von der Fraktur entfernt ein erstes Bohrloch angebracht. Nach Schneiden des Gewindes, Reposition, Auflegen der Platte und leichtem Anschrauben derselben mit der vorgesehenen ersten Schraube wird auf einer Distanz von etwa 18 mm von der Platte ein weiteres Bohrloch für die Spannvorrichtungsschraube angebracht und das Gewinde geschnitten. Anschließend wird der Plattenspanner aufgeschraubt, wobei der Haken in die Nute des äußersten Loches eingesetzt wird. Die Schraube des Spanners wird vorerst nur leicht angezogen, bis die Reposition einwandfrei erscheint. Jetzt werden im anderen Fragment die restlichen Plattenfixationsschrauben ein gedreht. Anschließend werden die Fragmente mittels eines Kardanschlüssels unter hohem Druck gesetzt. Wenn die Fragmente unter Druck liegen, wird die Reposition nochmals überprüft und es erfolgt dann das Anbringen der restlichen Schrauben. Am Schluß des Eingriffes wird die Spannschraube herausgedreht und der Spanner entfernt. In der Regel wird im letzten Loch der Kompressionsplatte noch eine Schraube eingesetzt, und zwar meist eine relativ kurze, damit die Elastizität des Knochens nicht zu abrupt unterbrochen wird.

Obgleich mit dieser Operationstechnik sehr gute Erfolge erreicht werden können, birgt sie noch beträchtliche Nachteile. Ein erster gewichtiger Nachteil besteht darin, daß man zur Fixation von Brüchen eine weitere Traumatisierung des Knochens durch die Einsetzung von Schrauben zur Fixierung der Kompressionsplatte und für die Spannvorrichtung vornehmen muß; in der Regel werden in jedes Fragment mehrere of drei bis fünf, teilweise sogar sieben und mehr Schrauben eingedreht. Diese weitere, therapiebedingte Traumatisierung schwächt durch relativ große Bohrlöcher zwangsläufig die Stabilität des Knochenrohres. Sie beeinträchtigt die Blutzirkulation in den Knochenfragmenten, die für eine primär angiogene Knochenheilung unerläßlich notwendig ist. Da Kompressionsplatten und Schrauben nach Abheilung der Fraktur wieder entfernt werden müssen, ergibt sich zu diesem Zeitpunkt eine nochmalige Traumatisierung des Knochens. Bei allen Patienten kann dies im weiteren Verlauf zu einer erhöhten erneuten Bruchanfälligkeit führen. Außerdem hat sich herausgestellt, daß durch nicht vermeidbare minimale Scher- und Torsionskräfte in den Gewindekanälen eine ständige Reizung und Belastung des Knochens erfolgt. Nach einiger Zeit wird der durch den Bohrvorgang geschädigte Knochen um die Schrauben herum durch ein bindegewebiges Lager ersetzt, in welchem die Schraube keinen festen Halt mehr finden kann. So hat man beispielsweise feststellen müssen, daß der anfängliche intrafragmentäre Druck im Laufe von 2 Monaten auf etwa die Hälfte zurückgehen kann. Die mechanischen Vorteile der Kompression und die Stabilität einer Osteosynthese werden naturgemäß beträchtlich reduziert, wenn der Druck während der ganzen Dauer der Bruchheilung zu schnell abnimmt. Außerdem gibt es noch einen weiteren Nachteil bei Verwendung der jetzt üblichen Kompressionsplatten und Schrauben, der in einer vermehrten Herauslösung von Ionen aus dem Metall besteht, was eine nichtinfektiöse Entzündung des Knochengewebes verursachen kann. Außerdem ist bekannt, daß aufgrund der natürlich vorkommenden Unregelmäßigkeiten der Knochenoberfläche die bisher verwendeten üblichen geraden, unmodulierten oder auch gewinkelten Platten nur eine Auflagefläche von ca. 30% am Knochen erreichen. Durch den Anpressdruck der Kompressionsplatte wird das Periost, das knochenernährende Gefäße führt, beschädigt. Die Folge ist eine zirkulärende Pororesierung, d.h. Schwächung des Knochenrohres, die zu den entscheidenden Nachteilen der Osteosynthese gehört.

Es besteht daher immer noch ein Bedürfnis danach, die bekannten Fixierungsmittel bei Frakturen und bei ihrem Einsatz in der Wiederherstellungschirurgie am Knochen so zu verändern, daß die obengeschilderten Nachteile vermieden werden.

Erfindungsgemäß wird nunmehr vorgeschlagen, statt der bisher üblichen Schrauben Nieten zur Befestigung der Kompressionsplatten, und zwar insbesondere Blindnieten einzusetzen. Außerdem werden Kompressionsplatten vorgeschlagen, die dadurch gekennzeichnet sind, daß die dem Knochen zugewandte Auflagefläche über Noppen oder Vorsprünge verfügt.

Die Verwendung von Nieten anstelle von Schrauben bietet eine Reihe von Vorteilen, denn dadurch ist es nicht mehr notwendig, in das Knochengewebe Bohrlöcher mit relativ großem Durchmesser wegen des notwendigen Gewindeganges einzudrehen, sondern der Druchmesser für die Einsatzlöcher der Nieten kann wesentlich kleiner gehalten werden. Die Nieten bestehen vorzugsweise aus möglichst inerten Material wie beispielsweise austenitischem Stahl oder aus Stahllegierungen mit Zusatz von Nickel, Kobald oder Crom. Der Nietenkern, gleichgültig aus welchem Material er besteht, kann kurz oberhalb des Abschlusses der Ummantelung mit einer Sollbruchstelle versehen sein. Beim Anlegen der Kompressionsplatte ist es dann möglich, daß man einfach nach der Befestigung den Kern des Nieten an der Sollbruchstelle abknipst. Gleichzeitig ergibt sich durch die Verwendung von Nieten eine einfachere Entfernung der Platte nach Abheilung, da die Nieten bei der Erstversorgung der Knochen zu einer sehr viel geringeren Traumatisierung des Knochengewebes führen und sie haben auch den Vorzug, daß durch die geringere Schädigung des Knochengewebes wegen Wegfalls des Schraubengewindes zwar ein bindegebiges Lager im Knochen entsteht, das aber bei weitem nicht Ausmaße und Durchmesser wie bei der Verwendung von Schrauben erreicht. Da außerdem die Kontaktflächen zwischen Metall und Knochengewebe deutlich verringert sind, reduziert sich auch die Anzahl von nichtinfektiösen Entzündungserscheinungen.

Erfindungsgemäß werden die Kompressionsplatten so ausgestaltet, daß die Auflagefläche nicht glatt ist, sondern über Noppen oder Vorsprünge verfügt, die als Auflage auf dem Knochen dienen. Auf diese Weise wird von vornherein davon ausgegangen, daß die Auflagefläche zwischen den Noppen der Platte, bezogen auf ihre Gesamtfläche, und dem Knochen nur etwa 30% beträgt. Dies ist ausreichend für eine hinreichende Fixation einer Fraktur und vermeidet die zusätzliche jatrogene Schädigugng des Periostes. Dadurch läßt sich die Durchblutung der Fragmente optimieren. Der Osteosynthese bedingte Schaden zählt bisher zu den Nachteilen der Schraubenosteosynthese mit relativ großen Bohrlöchern. Aufgrund der Entlastung des Knochens findet ein reduzierter interner Umbau der Kompakta des Knochens statt, d.h. geringere Porosierung/Spongesierung des Knochenmaterials. Da mit den erfindungsgemäß gestalteten Platten das Periost weitgehend geschont wird und eine zeitlich verzögerte Druckentlastung gegenüber der Schraubenosteosynthese eintritt resultiert eine wesentlich bessere und schnellere Heilung der Knochenwunde.

Im folgenden wird die Erfindung anhand der Zeichnungen näher erläutert:

Abb. 1 zeigt eine bisher übliche Kompressionsplatte mit Schrauben.

Abb. 2 zeigt einen erfindungsgemäßen Niet.

Abb. 3 zeigt eine erfindungsgemäße Kompressionsplatte.

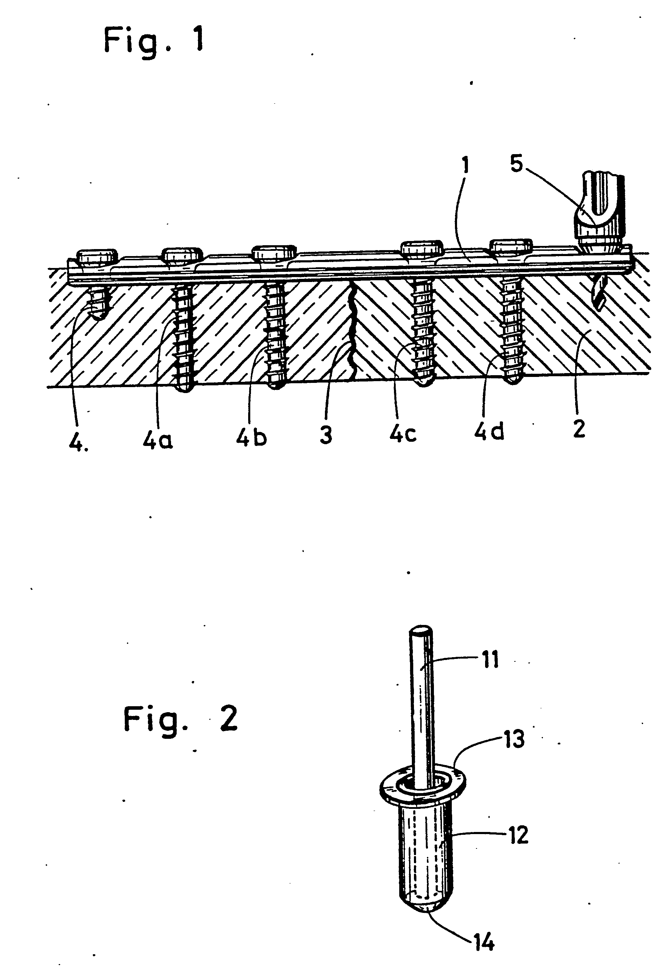

Wie aus Abb. 1 zu entnehmen, wird die Kompressionsplatte 1 zur Ruhestellung des Bruches 3 am Knochen 2 befestigt, und zwar durch Schrauben 4 - 4 d, wobei die am Ende der Platte sitzenden Schrauben wie Schraube 4 eine geringere Länge aufweisen als die übrigen Schrauben, um den Druck in etwa gleichmäßig auslaufen zu lassen. In der Darstellung ist ersichtlich, daß als Abschluß der Platte auf dem anderen Fragment der Gewindebohrer 5 gerade eingesetzt wird, um für die dann dort ebenfalls einzusetzende Schraube 4 die Eindrehung vorzubereiten.

Abb. 2 zeigt die anstelle der Schrauben einsetzbaren Nieten gemäß Erfindung. Die Niete besteht aus dem Nietenkern 11 mit der Ummantelung 12 und läuft am unteren Ende in den Nietenkopf 14 aus. Die Ummantelung ist als Abschluß mit einem Kragen 13 versehen. Die Kompression erfolgt nicht mehr über die Gewindegänge der Schraube im Knochenmark, sondern ausschließlich von außerhalb des dünnen Bohrloches von der Oberfläche der Gegenkorticalis aus.

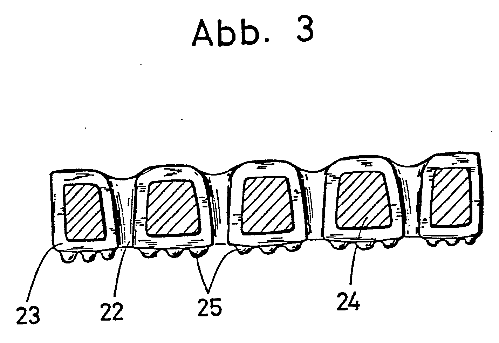

Abb. 3 zeigt einen Querschnitt durch eine erfindungsgemäße Kompressionsplatte 21 mit vorgeformten Bohrlöchern 22. Die Platte besteht aus einem Metallkern 24, der als flache Platte, als nebeneinanderliegende Drähte oder auch als Netz ausgebildet sein kann. Dieser Kern ist umgeben von einer Ummantelung 23 aus Metall oder einem anderen kompatiblen Material, die auf der dem Knochen zugewandten Auflegeseite mit Noppen oder Vorsprüngen 25 versehen. Selbstverständlich kann die Kompressionsplatte auch ohne speziellen Kern, sondern einstückig so ausgebildet sein, daß die Auflagefläche am Knochen wiederum Noppen oder Vorsprünge aufweist.

## Ansprüche

1. Osteosyntheseimplantate, dadurch gekennzeichnet, daß sie als Niet ausgebildet sind.

2. Implantate nach Anspruch 1, dadurch gekennzeichnet, daß sie als Blindniet ausgebildet sind.

3. Implantate nach Anspruch 1 oder 2, dadurch gekennzeichnet, daß der Kern oberhalb der Ummantelung mit einer Sollbruchstelle versehen ist.

4. Osteosyntheseimplantate, dadurch gekennzeichnet, daß sie als Kompressionsplatte ausgebildet sind, die auf der dem Knochen zugewandten Seite mit Noppen oder Vorsprüngen versehen sind.

5. Implantate nach Anspruch 4, dadurch gekennzeichnet, daß sie ein- oder mehrschichtig ausgebildet sind.

# Fig. 1

# Fig. 2

# Abb. 3

23      22      25      24